# EUROPEAN PATENT SPECIFICATION

(11) **EP 1 944 052 B1**
(45) Date of publication and mention of the grant of the patent: **02.09.2009**
(21) Application number: 07250131.5
(22) Date of filing: 12.01.2007
(51) Int. Cl.: A61M 25/06

(54) **Intravenous catheter introducing device**
Intravenöse Kathetereinführvorrichtung
Dispositif d'introduction intraveineuse d'un cathéter

(43) Date of publication of application: 16.07.2008
(73) Proprietor: Shue, Ming-Jeng, Hsi District, Taichung City (TW); Shue, Philip, Hsi District Taichung City (TW); Huang, Deborah, Taichung City (TW)
(72) Inventor: Shue, Ming-Jeng, Hsi District, Taichung City (TW); Shue, Philip, Hsi District Taichung City (TW); Huang, Deborah, Taichung City (TW)
(74) Representative: Lord, Hilton David

(56) References cited:
- EP-A- 0 812 601
- EP-A- 1 457 229
- EP-A- 1 514 568
- EP-A- 1 611 916
- US-A- 5 685 855

## Description

This invention relates to an intravenous catheter introducing device, more particularly to an intravenous catheter introducing device with a needle cannula which is retractable into a tubular plunger for safe disposal.

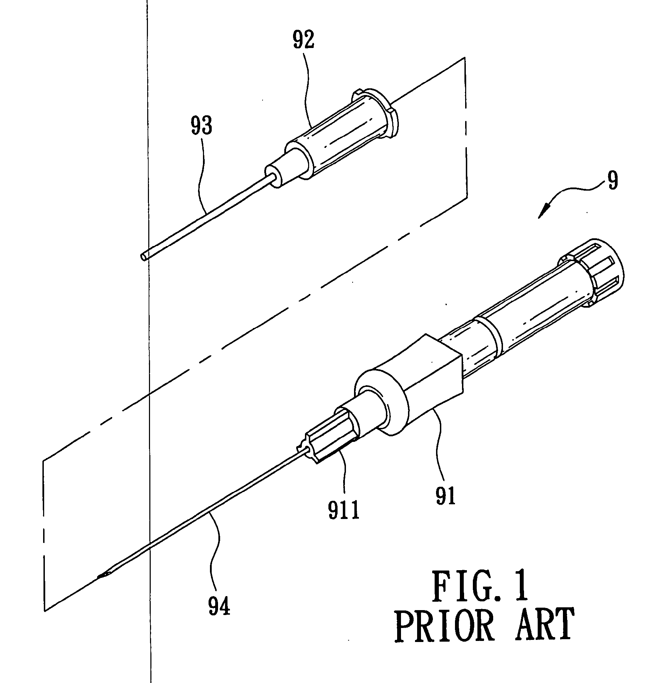

Intravenous catheter introducing devices are generally used to administer a medication fluid into or to draw blood from a patient's vein. Referring to Fig. 1, a conventional intravenous catheter introducing device 9 is shown to include a tubular needle seat 91 with a hub end 911, a needle cannula 94 secured to the hub end 911, a catheter hub 92 sleeved on the needle seat 91, and a flexible tubular catheter 93 secured to the catheter hub 92. In use, the catheter 93 and the needle cannula 94 are inserted into the patient's vein by a health care worker by piercing the patient's vein with a sharp tip of the needle cannula 94 which projects outwardly of the catheter 93. The health care worker then withdraws the needle cannula 94 from the catheter 93 with one hand and, at the same time, applies pressure to the patient's skin with the other hand, thereby leaving the catheter 93 in the patient's vein. Subsequently, a transfusion member (not shown) with the medication fluid or an empty barrel is connected to the catheter hub 92 for administering the medication fluid into the patient' s vein or for drawing blood. At this time, the exposed sharp tip of the used needle cannula 94 may create a danger of an accidental needle stick.

Moreover, the conventional intravenous catheter introducing device 9 is specifically not suitable for patients whose blood pressure is not sufficient to permit flow of blood therethrough, such as an emergency case, aged people, and pediatrics patients, and patients whose target vein is barely visible due to abundant adipose tissue, such as women and obese patients, since the health care worker will have difficulty determining whether the catheter 93 has been successfully introduced into the target vein, and may need to locate the vein by moving the needle cannula 94 in the skin of the patient, thereby complicating and prolonging the cannulation procedure and causing great discomfort to the patient.

Furthermore, although conventional self-retracting IV catheter introducers permit self-retraction of the used needle cannula into the syringe barrel after introduction of the catheter is completed, the blood in the barrel may be forced out of the barrel during the retraction of the used needle cannula so that blood contamination may occur.

A disposable syringe disclosed in EP 1,514,568 A1 includes a tubular plunger movable in a barrel relative to a needle seat that engages an inner surrounding barrel wall surface of the barrel by a resisting force. The plunger includes a plunger body and a coupling rod which is in frictional engagement with the plunger body and which has a central anchored area that is disposed to engage an anchoring portion of the needle seat by a holding force. As such, when the plunger body is moved to bring the central anchored area into engagement with the anchoring portion, the resisting force is overcome to permit disengagement of the needle seat from the barrel, and the coupling rod and the needle seat can be retracted into the plunger body by virtue of a biasing action of a biasing member.

An intravenous catheter introducing device disclosed in EP 1,611,916 A1 includes a needle hub inserted into and axially slidable relative to an inner barrel wall surface of a barrel . The needle hub holds a needle cannula and permits the cannula to extend forwardly of the barrel for ready use. A catheter connection assembly is detachably sleeved on a front smaller-diameter wall portion of the barrel and permits a tip end of the needle cannula to project forwardly of a tubular catheter of the assembly. A retaining hole is formed in a rear larger-diameter wall portion of the barrel such that a radially extending engaging peg is engageable in the retaining hole. Operation of an actuator mounted on the engaging peg can disengage the engaging peg from the retaining hole so as to permit axial movement of the needle hub for drawing the needle cannula within the barrel.

Although the disposable syringe and the intravenous catheter introducing device described above permit the needle cannula to be retracted into the barrel, the blood or medicine fluid in the barrel may be forced out of the barrel during the retraction so that contamination may occur. Moreover, no means is provided for facilitating flow of blood into the barrel to thereby enable the operator to conveniently observe and check the introduction of a catheter.

One object of the present invention is to provide an intravenous catheter introducing device which can be operated easily and safely to retract a used needle cannula with one hand, and which can prevent the blood from being forced out thereof.

Another object of the present invention is to provide an intravenous catheter introducing device which can facilitate flowing of the blood into the needle cannula to thereby enable the operator to conveniently observe and check the introduction of a catheter.

According to this invention, the intravenous catheter introducing device includes:
a needle cannula which has a front segment terminating at a tip end, and a rear connecting end opposite to the front segment along the axis in a longitudinal direction;
a tubular needle seat which includes a front hub portion that extends along the axis to terminate at a front end, and that is disposed to fix the rear connecting end therein, a gripped portion that extends from the front hub portion away from the front end, and a rear plug portion that extends from the gripped portion and distal from the front hub portion, and that has an internal duct extending through the gripped portion to be communicated with the rear connecting end;
a barrel which has an inner surrounding barrel surface defining a passage therein, the passage having rearward and forward openings, the inner surrounding barrel surface including a larger-diameter portion and a smaller-diameter portion which are disposed proximate to the rearward and forward openings, respectively, thelarger-diameter portion having a retaining area which is spaced apart from the smaller-diameter portion in the longitudinal direction;
a tubular grip member which, in a position of use, is disposed to hold, with a holding force, the gripped portion in a position of immovability along the axis relative to the retaining area;
a tubular plunger which is disposed to be movable in the passage along the larger-diameter portion, the plunger having a front opened endwall which is movable to abut against the grip member so as to place the tubular plunger in a pre-disposal position, a rear end wall which is disposed opposite to the front opened end wall, and which extends outwardly of the rearward opening so as to be manually operable, and an intermediate surrounding wall which is interposed between the front opened end wall and the rear end wall, and which defines an accommodation compartment;
a tubular receptacle which has a socket end, an air-permeable end, and a tubular wall segment interposed therebetween to confine a flashback chamber, wherein when the tubular grip member is in the position of use, the tubular receptacle is retained in the accommodation compartment by a first friction force, with the socket end and the air-permeable end respectively confronting the rear plug portion and the rear end wall to establish air communication between the internal duct and the flashback chamber, such that when the tubular plunger is in the pre-disposal position, the socket end is engaged with the rear plug portion, such that when the grip member is pushed forward by virtue of a forward movement of the front opened end wall against the holding force, the gripped portion is released from the grip member so as to permit axial movement of the gripped portion relative to the retaining area, and such that when the gripped portion is released from the grip member, a continued forward movement of the front opened end wall against the first friction force will result in movement of the tubular needle seat together with the tubular receptacle, through the engagement of the rear plug portion with the socket end, towards the rear end wall by virtue of a biasing force so as to place the tubular needle seat and the needle cannula in a disposal position, where the tip end of the needle cannula is retracted into the passage, and where the socket end is closer to the rear end wall than in the position of use;
a biasing member disposed to provide the biasing force;
a catheter hub which defines therein a duct that permits extension of the front segment of the needle cannula therethrough; and
a tubular catheter having a proximate segment which is inserted into the duct, and a distal segment which extends from the proximate segment and which surrounds and sheathes the front segment of the needle cannula while permitting the tip end to project forwardly of the distal segment for piercing a patient's skin.

Preferably, an air-permeable member which is made from a porous material is integrally formed with the air-permeable end so as to prevent blood from trickling out of the flashback chamber.

Preferably, the tubular plunger has an outlet which permits air communication between the accommodation compartment and the ambient air, and which is disposed downstream of the air permeable end.

Other features and advantages of the present invention will become apparent in the following detailed description of the preferred embodiments of the invention, with reference to the accompanying drawings, in which:
Fig. 1 is a perspective view of a conventional IV catheter introducing device;
Fig. 2 is an exploded sectional view of a first preferred embodiment of an intravenous catheter introducing device according to this invention;
Fig. 3 is a sectional view of the first preferred embodiment in a ready-to-use position;
Fig. 4 is a fragmentary sectional view of a portion of the first preferred embodiment;
Fig. 5 is a sectional view of the first preferred embodiment in a position of use;
Fig. 6 is a sectional view of the first preferred embodiment in a pre-disposal position;
Fig. 7 is a sectional view of the first preferred embodiment during an operation of retracting a needle cannula;
Fig. 8 is a sectional view of the first preferred embodiment in a retracted position;
Fig. 9 is a sectional view of the first preferred embodiment showing two outlets in a closed position;
Fig. 10 is a sectional view of the second preferred embodiment of an intravenous catheter introducing device according to this invention;
Fig. 11 is a sectional view of the second preferred embodiment in a retracted position;
Fig. 12 is a sectional view of the third preferred embodiment of an intravenous catheter introducing device according to this invention;
Fig. 13 is a sectional view of the third preferred embodiment in a retracted position; and
Figs. 14 to 18 respectively are sectional view of the fourth to eighth preferred embodiments of an intravenous catheter introducing device according to this invention.

Before the present invention is described in greater detail, it should be noted that same reference numerals have been used to denote like elements throughout the specification.

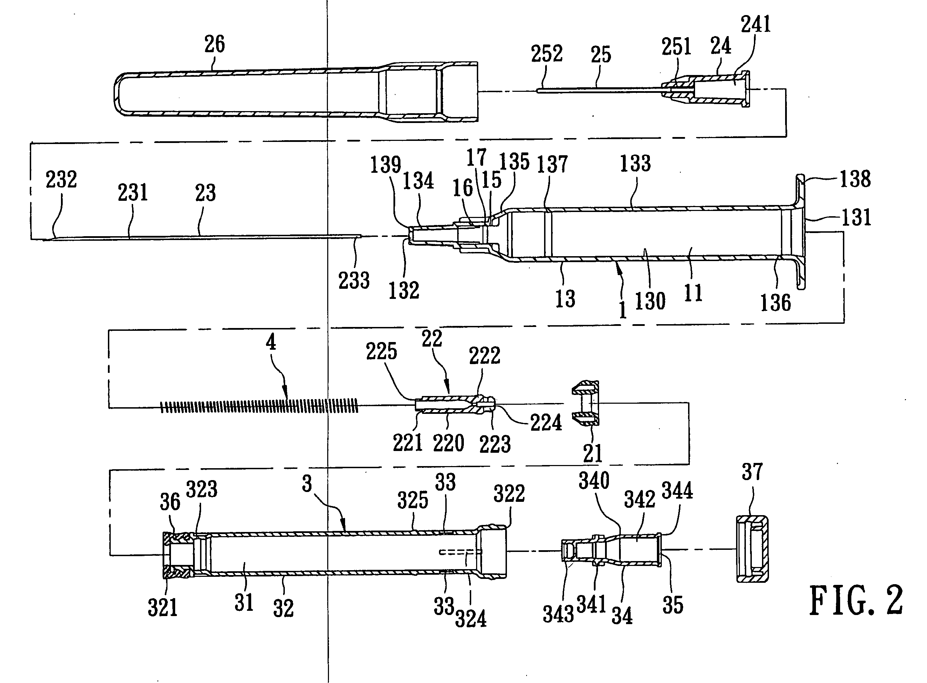

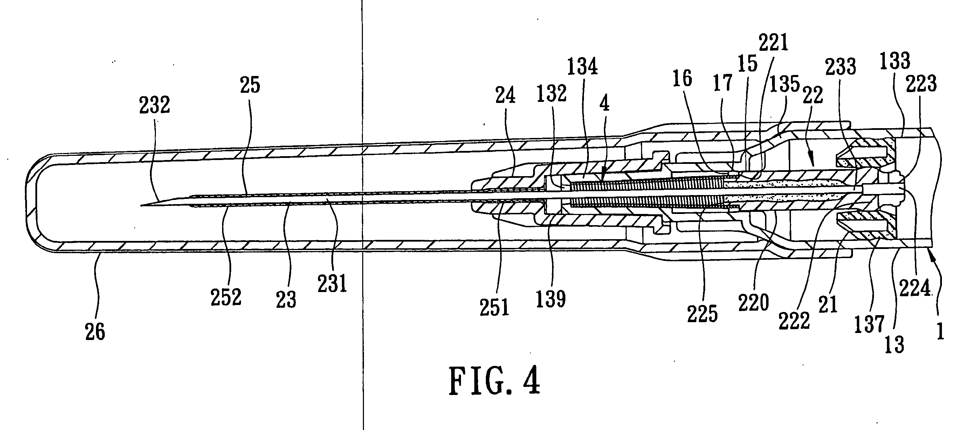

Referring to Figs. 2 to 4, the first preferred embodiment of an intravenous catheter introducing device according to the present invention is shown to comprise a barrel 1, a needle cannula 23, a tubular needle seat 22, a tubular grip member 21, a tubular plunger 3, a tubular receptacle 34, a biasing member 4, a catheter hub 24, a tubular catheter 25, and a tip protector 26.

The barrel 1 has a surrounding wall 13 surrounding an axis. The surrounding wall 13 has an inner surrounding barrel surface 130 which defines a passage 11 therein. The passage 11 has rearward and forward openings 131,132 which are opposite to each other in a longitudinal direction along the axis. The inner surrounding barrel surface 130 includes a larger-diameter portion 133 and a smaller-diameter portion 134 which are disposed proximate to the rearward and forward openings 131,132, respectively, and a shoulder 135 which is interposed therebetween. The larger-diameter portion 133 has a projecting retaining area 137 which is spaced apart from the smaller-diameter portion 134 in the longitudinal direction. The smaller-diameter portion 134 includes an entry region 15, a transit region 17 which extends from the entry region 15 forwardly, and an inner annular abutment surface 16 which confronts rearwardly. The barrel 1 further has a finger flange 138 disposed proximate to the rearward opening 131.

The needle cannula 23 has a front segment 231 terminating at a tip end 232, and a rear connecting end 233 opposite to the front segment 231 along the axis.

The tubular needle seat 22 includes a front hub portion 220 which extends along the axis to terminate at a front end 225 to be surrounded by the entry region 15, and which is disposed to fix the rear connecting end 233 of the needle cannula 23 therein, a gripped portion 222 which extends from the front hub portion 220 away from the front end 225, and a rear plug portion 223 which extends from the gripped portion 222 and distal from the front hub portion 220 and which has an internal duct 224 extending through the gripped portion 222 along the axis to be communicated with the rear connecting end 233 of the needle cannula 23. The front hub portion 220 has a shoulder surface 221 which is disposed rearwardly of the front end 225, and which is spaced apart from the inner annular abutment surface 16 by the transit region 17.

The tubular grip member 21 is retained at the retaining area 137 of the barrel 1 in a position of use so as to hold the gripped portion 222 of the needle seat 22 in a position of immovability along the axis relative to the retaining area 137 by virtue of a holding force. To be specific, the tubular grip member 21 is disposed in retaining engagement with the projecting retaining area 137 of the larger-diameter portion 133, and in gripping engagement with the gripped portion 222 of the needle seat 22 by second and third friction forces, respectively, which cooperate in radial directions to serve as the holding force.

The tubular plunger 3 is disposed to be movable in the passage 11 along the larger-diameter portion 130. The plunger 3 has a front opened end wall 321, a rear end wall 322 which is opposite to the front opened end wall 321 and which extends outwardly of the rearward opening 131 so as to be manually operable, and an intermediate surrounding wall 32 which is interposed between the front opened end wall 321 and the rear end wall 322 and which defines an accommodation compartment 31. The intermediate surrounding wall 32 has a plurality of ribs 324 which are formed on an outer surface thereof and adjacent to the rear end wall 322.

The tubular plunger 3 has two outlets 33 which communicate the accommodation compartment 31 with the ambient air, and which are disposed downstream of the air permeable end 344. In this embodiment, the outlets 33 are formed in the intermediate surrounding wall 32 adjacent to the rear end wall 322 to facilitate closing by a user's finger when the user grips and moves the tubular plunger 3.

The tubular plunger 3 further has a deformable sealing member 36 which is configured to surround the front opened end wall 321, and which is in air-tight sliding engagement with the larger-diameter portion 130.

The tubular receptacle 34 has a socket end 343 and an air-permeable end 344 spaced apart from each other in the longitudinal direction, and a tubular wall segment 340 interposed therebetween to confine a flashback chamber 342. In the position of use, the tubular receptacle 34 is retained in the accommodation compartment 31 by a first friction force generated between protrusion and recess portions 341,323 such that the socket end 343 and the air-permeable end 344 respectively confront the rear plug portion 223 and the rear end wall 322 to establish an air communication between the internal duct 224 and the flashback chamber 342. An air-permeable member 35 is integrally formed with the air-permeable end 344, and is made from a porous material.

The rear end wall 322 of the tubular plunger 3 defines an access opening for insertion of the tubular receptacle 34 into the accommodation compartment 31 therethrough. An end cap 37 is detachably mounted to the rear end wall 322 to close the access opening.

The barrel 1 has a forward end wall 139 which defines the forward opening 132. The biasing member 4 is in the form of a coil spring 4 which surrounds the front segment 231 of the needle cannula 23, and which is compressed between the forward end wall 139 and the shoulder surface 221 of the needle seat 22 to provide a biasing force to move the needle seat 22 rearwardly.

The catheter hub 24 is detachably sleeved on the surrounding wall 13 of the barrel 1, and defines therein a duct 241 that permits extension of the front segment 231 of the needle cannula 23 therethrough.

The tubular catheter 25 has a proximate segment 251 which is inserted into the duct 241, and a distal segment 252 which extends from the proximate segment 251 and which surrounds and sheathes the front segment 231 of the needle cannula 23 while permitting the tip end 232 to project forwardly of the distal segment 252 for piercing a patient's skin.

The tip protector 26 is removably sleeved on the surrounding wall 13 for shielding the needle cannula 23.

In the position of use, the front hub portion 220 of the needle seat 22 is retained at the retaining area 137 by the holding force (i.e., the second and third friction forces), and the inner annular abutment surface 16 is spaced apart from the shoulder surface 221 by the transit region 17.

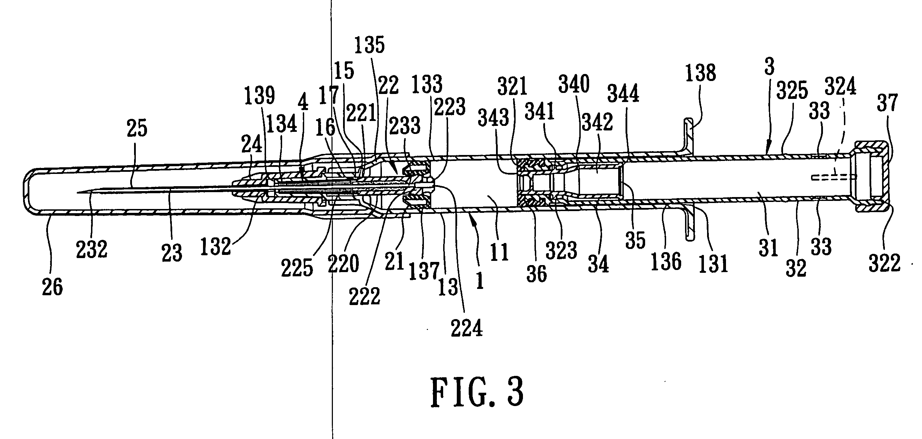

Referring to Figs. 3, 4 and 5, in an IV introducing stroke, the tip protector 26 is removed first to expose the tip end 232 of the needle cannula 23. The operator grips the surrounding wall 13 of the barrel 1 and pierces the patient's vein with the tip end 232 so as to introduce the tubular catheter 25 into the vein. The blood flowing into the passage 11 is visible from the surrounding wall 13 so that the operator can check whether the needle cannula 23 has been inserted properly into the vein. The operator can then separate the catheter hub 24 from the barrel 1, and the IV introducing operation is completed.

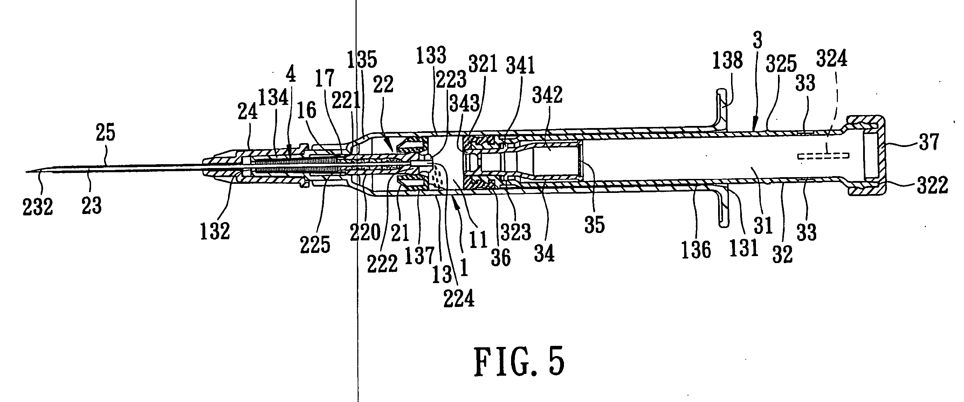

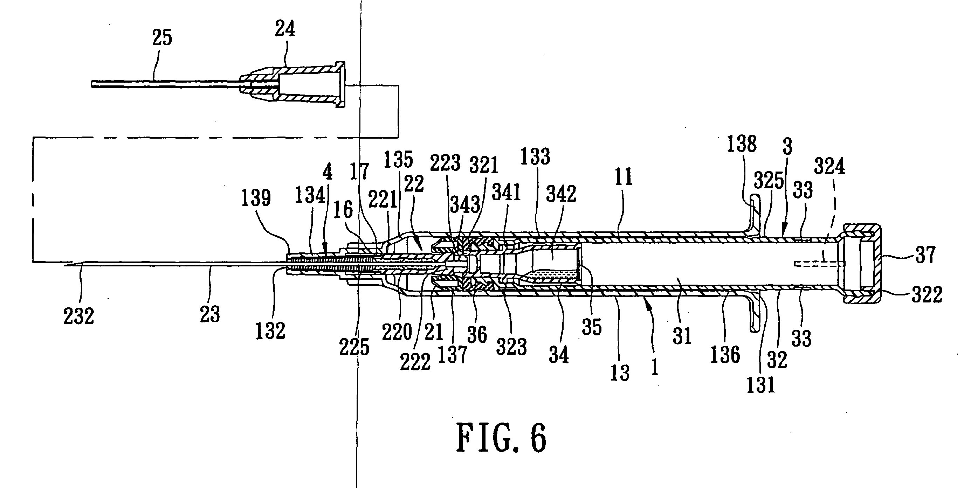

Referring to Figs. 5 and 6, since the passage 11 is in air communication with the accommodation compartment 31 via the flashback chamber 342 so as to be communicated with the ambient air through the outlets 33, after the IV introducing stroke, the operator canpress the tubularplunger 4 forwardly so as to move the deformable sealing member 36 to abut against the grip member 21 to thereby place the tubular plunger 3 in a pre-disposal position. At this time, the blood in the passage 11 can flow into the flashback chamber 342 due to air communication of the passage 11 with the ambient air, and the air-permeable member 35 can prevent the blood from trickling out of the flashback chamber 342. Thus, the blood will not be forced out of the barrel 1 during forward pressing of the tubular plunger 3.

Subsequently, the socket end 343 is engaged with the rear plug portion 223. When the grip member 21 is pushed forward by virtue of a forward movement of the tubular plunger 3 against the holding force, the grip member 21 is moved to disengage from the retaining region 137 so that the second friction force disappears, and the shoulder surface 221 of the needle seat 22 is moved to pass from the entry region 15 to the transit region 17 to abut against the inner annular abutment surface 16. Then, by virtue of the abutment of the shoulder surface 221 against the inner annular abutment surface 16, the gripped portion 222 is released from the grip member 21 so that the third friction force disappears. The time lag between the releasing of the second and third friction forces can result in a smooth operation of the tubular plunger 3. At the same time, by virtue of a counteracting effect of the forward end wall 139 of the barrel 1 and the coil spring 4, the protrusion portion 341 of the receptacle 34 is moved to disengage from the recess portion 323 in the plunger 3 so as to permit releasing of the coil spring 4.

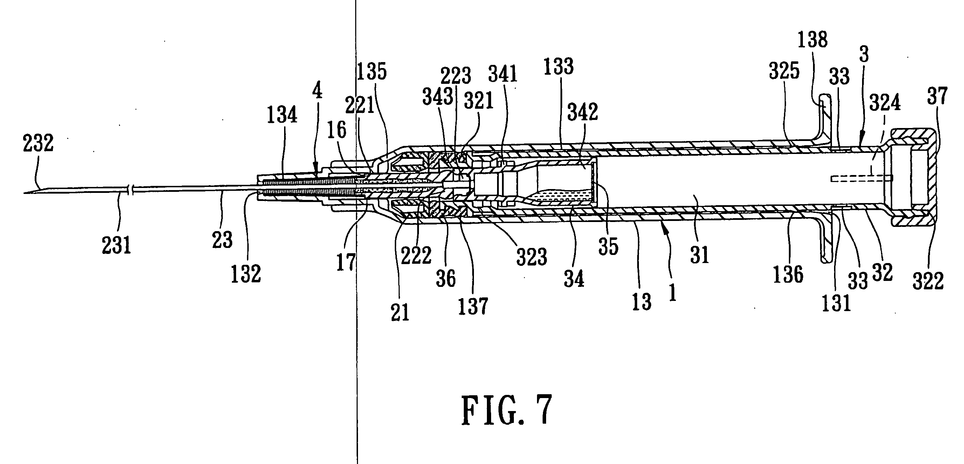

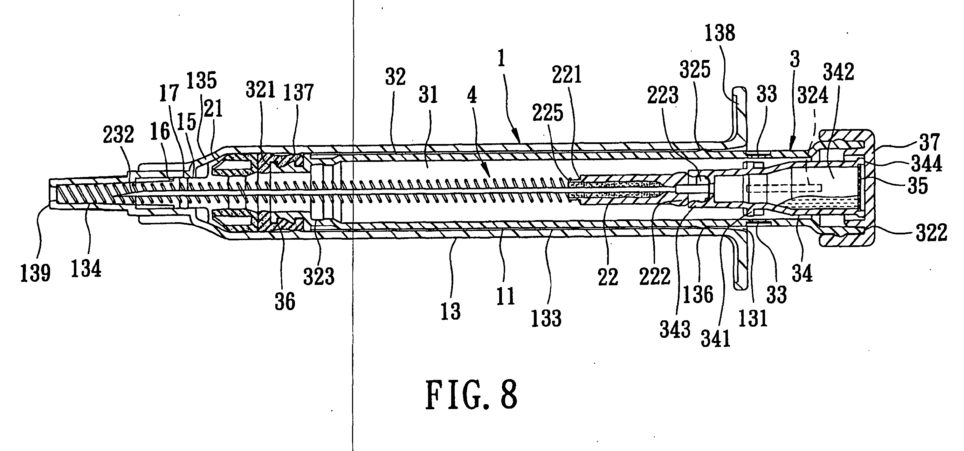

Therefore, as shown in Fig. 7, through the engagement of the rear plug portion 223 with the socket end 343, a rearward movement of the needle seat 22 will, by virtue of the biasing force of the coil spring 4, force the tubular receptacle 34 to move towards the rear end wall 322 to place the tubular needle seat 22 and the needle cannula 23 in a disposal position, as shown in Fig. 8, where the tip end 232 of the needle cannula 23 is retracted into the passage 11, and where the socket end 343 is closer to the rear end wall 322 than in the position of use.

It is noted that when the tubular plunger 3 is pressed to place the tubular needle seat 22 and the needle cannula 23 in the disposal position, the tubular plunger 3 is retained at the larger-diameter portion 133 through snug engagement between an annular recess 136 and an annular projection 325 so as to prevent rearward pulling of the tubular plunger 3 for reuse. Moreover, the forward pressing of the tubular plunger 3 can be limited by the ribs 324 that abut against the barrel 1 at the rearward opening 131 so as to prevent excess forward movement of the tubular plunger 3, which may cause the grip member 21 to deform and interfere with the rearward movement of the needle seat 22.

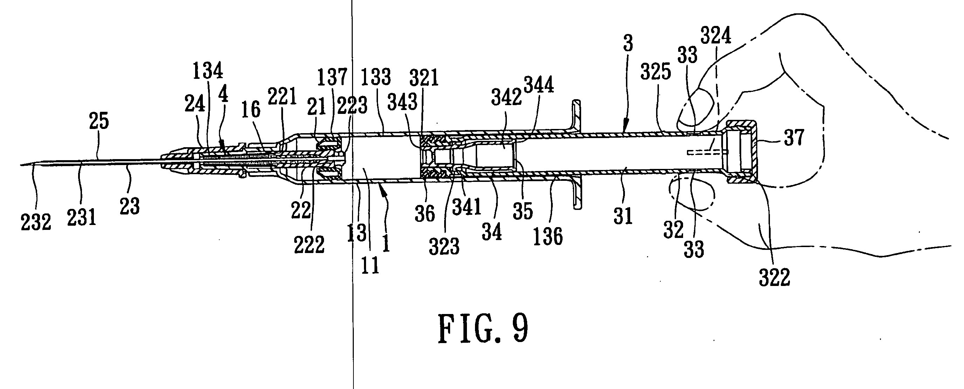

Referring to Fig. 9, during the IV introducing operation, if blood is not observed in the passage 11, the operator can hold the tubular plunger 3 and close the outlets 33 with his/her thumb and index finger to interrupt air communication between the passage 11 and the ambient air. Then the operation can pull the tubular plunger 3 rearwardly so as to generate a reduced pressure in the passage 11 to thereby facilitate flow of blood into the passage 11. Thus, the operator can easily check whether the tubular catheter 25 has been successfully introduced into a target vein of the patient.

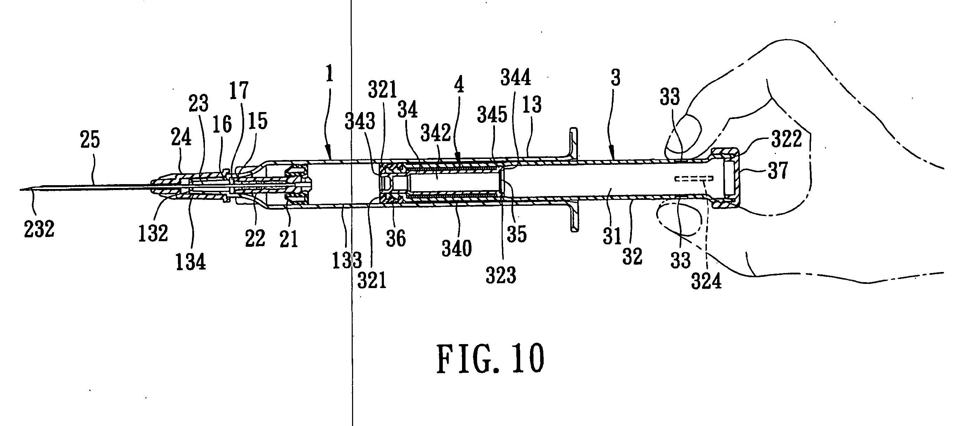

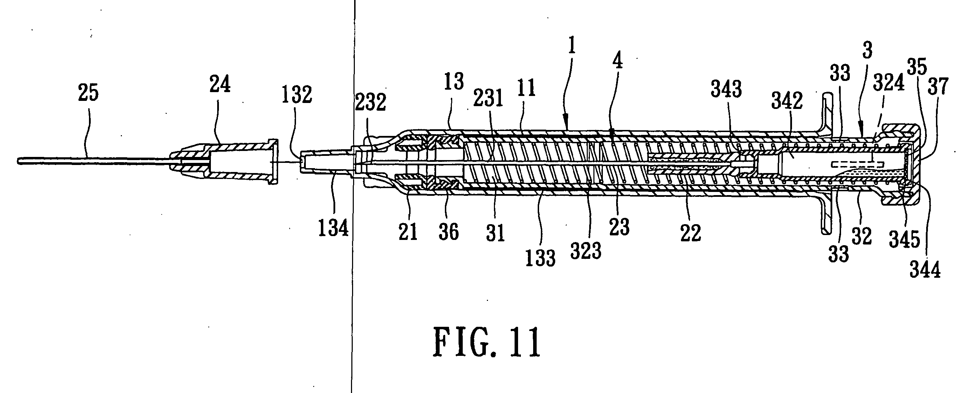

Referring to Figs. 10 and 11, the second preferred embodiment of an intravenous catheter introducing device according to this invention is shown to be similar to the first preferred embodiment in construction. The difference resides in that the tubular wall segment 340 of the tubular receptacle 34 has an outer annular abutment surface 345 adjacent to the air-permeable end 344. The biasing member 4 is in the form of a coil spring 4 which surrounds the tubular wall segment 340 and which is compressed between the front opened end wall 321 and the outer annular abutment surface 345. Moreover, the outer annular abutment surface 345 is retained at the recess portion 323 of the tubular plunger 3 to generate the first friction force.

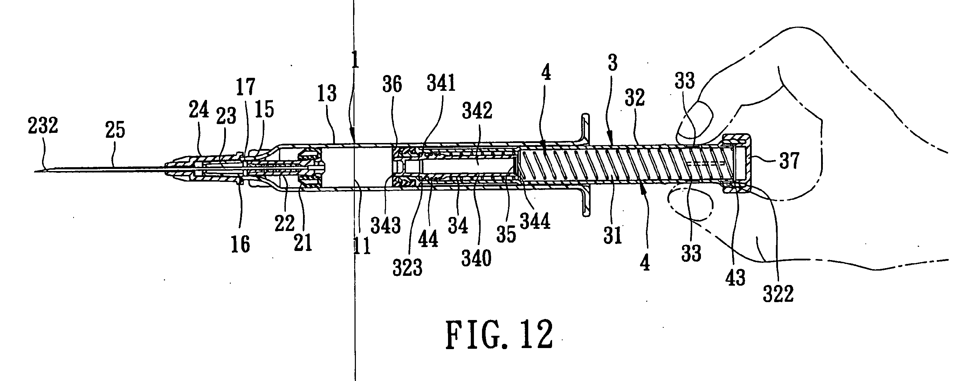

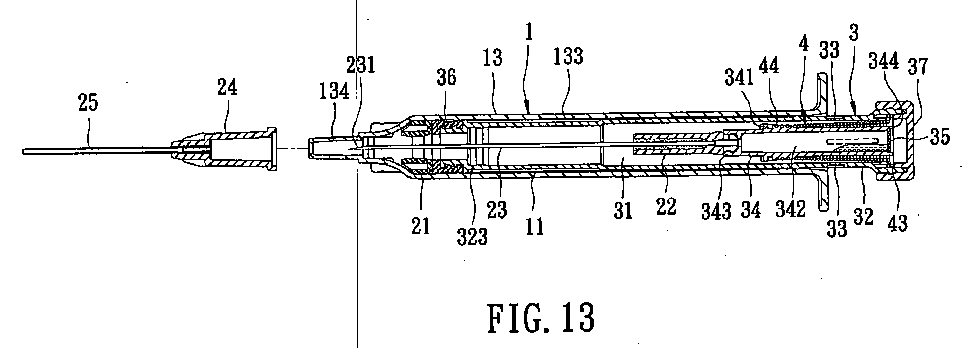

Referring to Figs. 12 and 13, the third preferred embodiment of an intravenous catheter introducing device according to this invention is shown to be similar to the first preferred embodiment in construction. The difference resides in that the biasing member 4 is in the form of a coil spring 4 which is received in the accommodation compartment 31 of the tubular plunger 3 and which has a secured end 43 that is secured to the intermediate surrounding wall 32 adjacent to the rear end wall 322, and a tensed end 44 that is secured to the tubular wall segment 340 of the tubular receptacle 34 so as to remain tensed in the position of use.

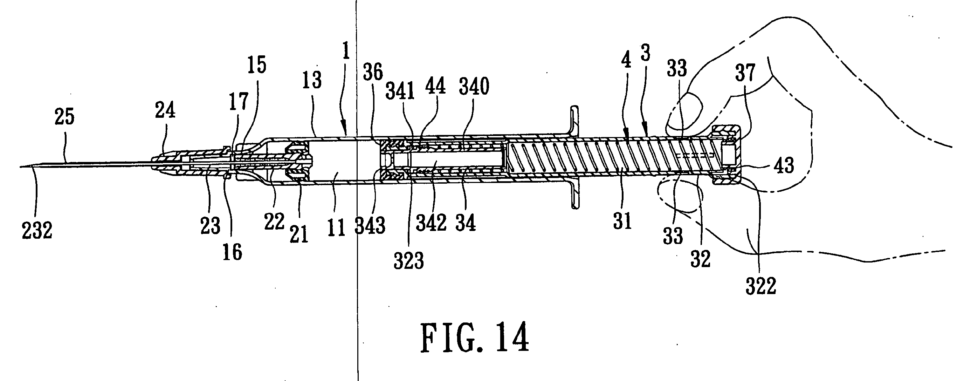

Referring to Fig. 14, the fourth preferred embodiment of an intravenous catheter introducing device according to this invention is shown to be similar to the third preferred embodiment in construction. The difference resides in that the biasing member 4 has a secured end 43 which is secured to the end cap 37.

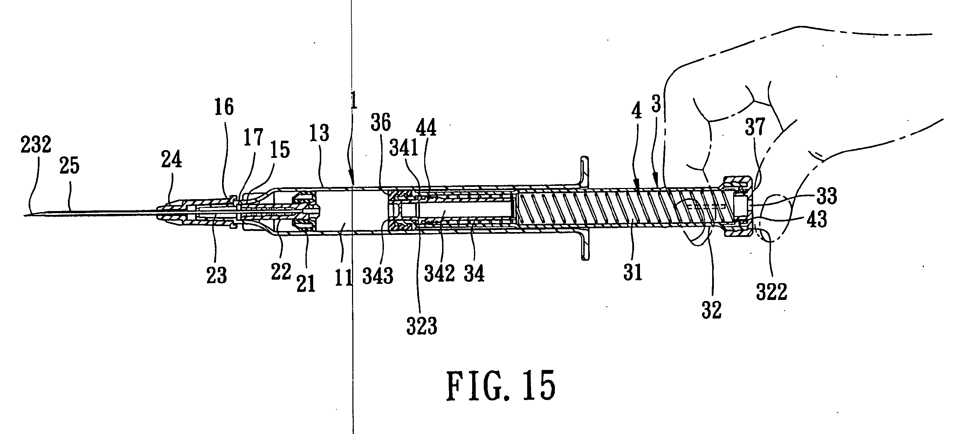

Referring to Fig. 15, the fifth preferred embodiment of an intravenous catheter introducing device according to this invention is shown to be similar to the third preferred embodiment in construction. The difference resides in that the outlet 33 is formed in the end cap 37 to facilitate closing of the outlet 33 by the operator.

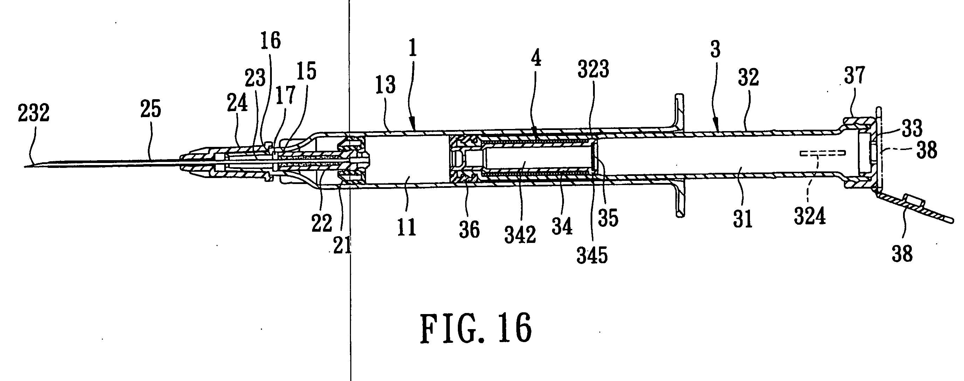

Referring to Fig. 16, the sixth preferred embodiment of an intravenous catheter introducing device according to this invention is shown to be similar to the second preferred embodiment in construction. The difference resides in that the outlet 33 is formed in the end cap 37, and a cover plate 38 is disposed on the tubular plunger 3, and is movable relative to the tubular plunger 3 between closing and opening positions, where the cover plate 38 engages with and disengages from the end cap 37 to close and open the outlet 33, respectively.

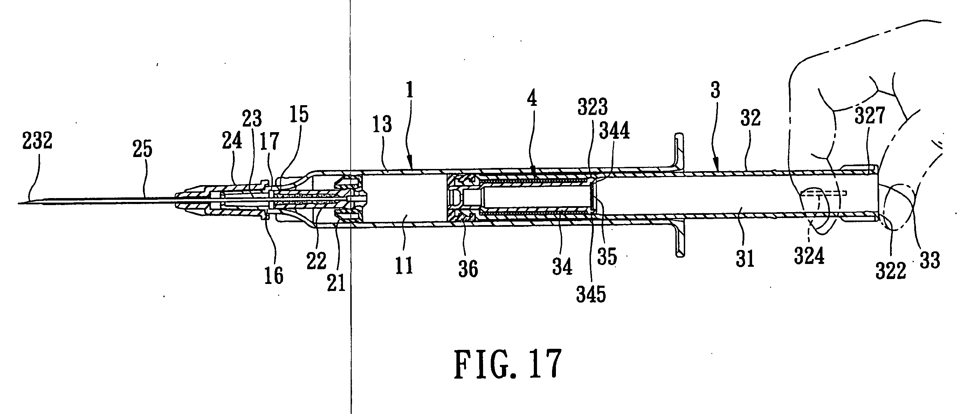

Referring to Fig. 17, the seventh preferred embodiment of an intravenous catheter introducing device according to this invention is shown to be similar to the second preferred embodiment in construction. The difference resides in that the outlet 33 is formed in the rear end wall 322 of the tubular plunger 3, and the intermediate surrounding wall 32 of the tubular plunger 3 has an annular barrier 327 formed adjacent to the rear end wall 322 such that the air-permeable end 344 of the tubular receptacle 34 is stopped by the annular barrier 327 when the tubular receptacle 34 is moved to the disposal position.

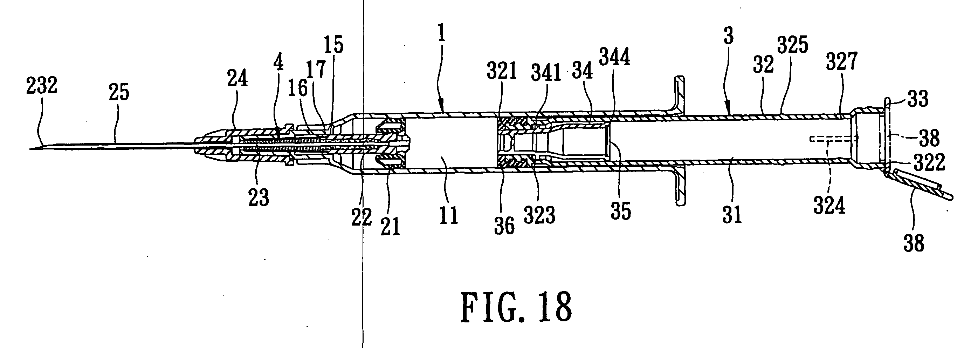

Referring to Fig. 18, the eighth preferred embodiment of an intravenous catheter introducing device according to this invention is shown to be similar to the first preferred embodiment in construction. The difference resides in that the outlet 33 is formed in the rear end wall 322 of the tubular plunger 3, and the intermediate surrounding wall 32 of the tubular plunger 3 has an annular barrier 327 formed adjacent to the rear end wall 322 such that the air-permeable end 344 of the tubular receptacle 34 is stopped by the annular barrier 327 when the tubular receptacle 34 is moved to the disposal position. In addition, a cover plate 38 is disposed on the tubular plunger 3 and is movable relative to the tubular plunger 3 between closing and opening positions, where the cover plate 38 engages and disengages from the rear end wall 322 to close and open the outlet 33, respectively.

As illustrated, according to the intravenous catheter introducing device of this invention, the used needle cannula 23 can be retracted into the passage 11 of the barrel 1 to thereby avoid occurrence of an accidental needle stick. Further, during the forward pressing of the tubular plunger 3 to retract the needle cannula 23, since the passage 11 is air communicated with the ambient air, the blood in the passage 11 may flow into the flashback chamber 342, and the air-permeable member 35 can prevent the blood from trickling out of the flashback chamber 342. Thus, the blood will not be forced out of the barrel 1. Moreover, during the IV introducing operation, once blood is not observed in the passage 11, the operator can close the outlet (s) 33 and pull the tubular plunger 3 rearwardly to generate a reduced pressure in the passage 11 to thereby facilitate flow of blood into the passage 11. Thus, the operator can easily check whether the tubular catheter 25 has been successfully introduced into a target vein of the patient.

## Claims

1. An intravenous catheter introducing device comprising:
a needle cannula (23) having a front segment (231) terminating at a tip end (232), and a rear connecting end (233) opposite to said front segment (231) along the axis in a longitudinal direction;
a tubular needle seat (22) which includes a front hub portion (220) that extends along the axis to terminate at a front end (225), and that is disposed to fix said rear connecting end (233) therein, a gripped portion (222) that extends from said front hub portion (220) away from said front end (225), and a rear plug portion (223) that extends from said gripped portion (222) and distal from said front hub portion (220), and that has an internal duct (224) extending through said gripped portion (222) along the axis to be communicated with said rear connecting end (233);
a barrel (1) having an inner surrounding barrel surface (130) which surrounds the axis and which defines a passage (11) therein, said passage (11) having rearward and forward openings (131,132) which are opposite to each other in the longitudinal direction, said inner surrounding barrel surface (130) including a larger-diameter portion (133) and a smaller-diameter portion (134) which are disposed proximate to said rearward and forward openings (131, 132), respectively, said larger-diameter portion (133) having a retaining area (137) which is spaced apart from said smaller-diameter portion (134) in the longitudinal direction;
a tubular grip member (21) which, in a position of use, is disposed to hold, with a holding force, said gripped portion (222) in a position of immovability along the axis relative to said retaining area (137); and
a tubular plunger (3) which is disposed to be movable in said passage (11) along said larger-diameter portion (130),
said plunger (3) has a front opened end wall (321) which is movable to abut against said grip member (21) so as to place said tubular plunger (3) in a pre-disposal position, a rear end wall (322) which is disposed opposite to said front opened end wall (321), and which extends outwardly of said rearward opening (131) so as to be manually operable, and an intermediate surrounding wall (32) which is interposed between said front opened end wall (321) and said rear end wall (322), and which defines an accommodation comportment (31);
said intravenous catheter introducing device further comprising:
a tubular receptacle (34) which has a socket end (343) and an air-permeable end (344) spaced apart from each other in the longitudinal direction, and a tubular wall segment (340) interposed between said socket end (343) and said air-permeable end (344) to confine a flashback chamber (342), wherein, when said tubular grip member (21) is in the position of use, said tubular.receptacle (34) is retained in said accommodation compartment (31) by a first friction force, with said socket end (343) and said air-permeable end (344) respectively confronting said rear plugportion (223) and said rear end wall (322) to establish air communication between said internal duct (224) and said flashback chamber (342),
such that when said tubular plunger (3) is in the pre-disposal position, said socket end (343) is engaged with said rear plug portion (223),
such that when said grip member (21) is pushed forward by virtue of a forward movement of said front opened end wall (321) against the holding force, said gripped portion (222) is released from said grip member (21) to permit axial movement of said gripped portion (222) relative to said retaining area (137), and
such that when said gripped portion (222) is released from said grip member (21), a continued forward movement of said front opened end wall (321) against the first friction force will result in movement of said needle seat (22) together with said tubular receptacle (34), through the engagement of said rear plug portion (223) with said socket end (343), towards said rear end wall (322) by virtue of a biasing force so as to place said needle seat (22) and said needle cannula (23) in a disposal position, where said tip end (232) of said needle cannula (23) is retracted into said passage (11), and where said socket end (343) is closer to said rear end wall (322) than in the position of use; and
a biasing member (4) disposed to provide said biasing force.

2. The intravenous catheter introducing device of Claim 1, **characterized in that** said smaller-diameter portion (134) includes an entry region (15) which is configured to surround said front hub portion (220), and a transit region (17) which extends from said entry region (15) towards said forward opening (132), said tubular grip member (21) being disposed to be in retaining engagement with said retaining area (137) of said larger-diameter portion (133), and in gripping engagement with said gripped portion (222) of said needle seat (22) by second and third friction forces, respectively, which cooperate in radial directions to serve as the holding force, such that once an axial movement of said gripped portion (222) commences relative to said retaining area (137) by virtue of the forward movement of said grip member (21) and said front opened end wall (321), said front hub portion (220) is moved to pass from said entry region (15) to said transit region (17) so as to permit said tubular grip member (21) to be disengaged from said retaining area (137) before disengagement of said gripped portion (222) from said tubular grip member (21) in the pre-disposal position.

3. The intravenous catheter introducing device of Claim 2, **characterized in that** said front hub portion (220) of said needle seat (22) has a shoulder surface (221) which is disposed rearwardly of said front end (225), and which faces towards said forward opening (132), said smaller-diameter portion (134) further including an inner annular abutment surface (16) which is spaced apart from said shoulder surface (221) by said transit region (17) such that by virtue of abutment of said shoulder surface (221) against said inner annular abutment surface (16), forward movement of said grip member (21) and said front opened end wall (321) results in release of said gripped portion (222) from said grip member (21) against the third friction force.

4. The intravenous catheter introducing device of Claim 3, **characterized in that** said barrel (1) has a forward end wall (139) which defines said forward opening (132), said biasing member (4) being in the form of a coil spring (4) which surrounds said front segment (231) of said needle cannula (23), and which is compressed between said forward end wall (139) and said shoulder surface (221) of said needle seat (22).

5. The intravenous catheter introducing device of Claim 1, **characterized in that** said tubular wall segment (340) of said tubular receptacle (34) has an outer annular abutment surface (345) adjacent to said air-permeable end (344), said biasing member (4) being in the form of a coil spring (4) which surrounds said tubular wall segment (340) and which is compressed between said front opened end wall (321) and said outer annular abutment surface (345).

6. The intravenous catheter introducing device of Claim 1, **characterized in that** said biasing member (4) is in the form of a coil spring (4) which is received in said accommodation compartment (31) and which has a secured end (43) that is secured to said intermediate surrounding wall (32) adjacent to said rear end wall (322), and a tensed end (44) that is secured to said tubular wall segment (340) of said tubular receptacle (34) so as to remain tensed in the position of use.

7. The intravenous catheter introducing device of Claim 1, **characterized in that** said rear end wall (322) of said tubular plunger (3) defines an access opening for insertion of said tubular receptacle (34) into said accommodation compartment (31), said device further comprising an end cap (37) which is detachably mounted to said rear end wall (322) to close said access opening, said biasing member (4) being in the form of a coil spring (4) which is received in said accommodation compartment (31) and which has a secured end (43) that is secured to said end cap (37), and a tensed end (44) that is secured to said tubular wall segment (340) of said tubular receptacle (34) so as to remain tensed in the position of use.

8. The intravenous catheter introducing device of Claim 1, further **characterized by** an air-permeable member (35) which is made from a porous material, and which is integrally formed with said air-permeable end (344) so as to prevent blood from trickling out of said flashback chamber (342).

9. The intravenous catheter introducing device of Claim 1, **characterized in that** said tubular plunger (3) has a deformable sealing member (36) which is configured to surround said front opened end wall (321), and which is in air-tight sliding engagement with said larger-diameter portion (130).

10. The intravenous catheter introducing device of Claim 1, **characterized in that** said tubular plunger (3) has an outlet (33) that communicates said accommodation compartment (31) with the ambient air, and that is disposed downstream of said air permeable end (344).

11. The intravenous catheter introducing device of Claim 10, **characterized in that** said outlet (33) is formed in said intermediate surrounding wall (32) adjacent to said rear end wall (322) to facilitate closing by a user's finger when the user grips and moves said tubular plunger (3).

12. The intravenous catheter introducing device of Claim 10, **characterized in that** said rear end wall (322) of said tubular plunger (3) defines an access opening for insertion of said tubular receptacle (34) into said accommodation compartment (31), said device further comprising an end cap (37) which is detachably mounted to said rear end wall (322) to close said access opening, said outlet (33) being formed in said end cap (37).

13. The intravenous catheter introducing device of Claim 10, **characterized in that** said outlet (33) is formed in said rear end wall (322) of said tubular plunger (3).

14. The intravenous catheter introducing device of Claim 13, further **characterized by** a cover plate (38) which is disposed on said tubular plunger (3) and which is movable relative to said tubular plunger (3) between closing and opening positions, where said cover plate (38) engages and disengages from said rear end wall (322) to close and open said outlet (33), respectively.

15. The intravenous catheter introducing device of Claim 13, **characterized in that** said intermediate surrounding wall (32) of said tubular plunger (3) has an annular barrier (327) formed adjacent to said rear end wall (322) such that said air-permeable end (344) of said tubular receptacle (34) is stopped by said annular barrier (327) when said tubular receptacle (34) is moved to the disposal position.

16. The intravenous catheter introducing device of Claim 1, further **characterized by**:
a catheter hub (24) which defines therein a duct (241) that permits extension of said front segment (231) of said needle cannula (23) therethrough; and
a tubular catheter (25) having a proximate segment (251) which is inserted into said duct (241), and a distal segment (252) which extends from said proximate segment (251) and which surrounds and sheathes said front segment (231) of said needle cannula (23) while permitting said tip end (232) to project forwardly of said distal segment (252) for piercing a patient's skin.

17. The intravenous catheter introducing device of Claim 1, **characterized in that** said tubular plunger (3) and said larger-diameter portion (133) respectively have an annular projection (325) and an annular recess (136) which are configured such that once said tubular plunger (3) is placed in the pre-disposal position, said annular projection (325) and said annular recess (136) are snugly engaged with each other so as to prevent rearward pulling of said tubular plunger (3) for reuse.

18. The intravenous catheter introducing device of Claim 1, **characterized in that** said intermediate surrounding wall (32) of said tubular plunger (3) has a plurality of ribs (324) which are formed on an outer surface thereof and adjacent to said rear end wall (322) and which are disposed to abut against said barrel (1) at said rearward opening (131) when said tubular plunger (3) is in the disposal position.

## Patentansprüche

1. Intravenöse Katheter-Einführungseinrichtung, die Folgendes umfasst:
eine Nadelkanüle (23), die ein vorderes Segment (231), das an einem Spitzenende (232) endet, und ein hinteres Verbindungsende (233), längs der Achse in einer Längsrichtung entgegengesetzt zu dem vorderen Segment (231), hat,
einen röhrenförmigen Nadelsitz (22), der einen vorderen Verbindungsstück-Abschnitt (220), der sich längs der Achse erstreckt, um an einem vorderen Ende (225) zu enden, und der dafür angeordnet ist, das hintere Verbindungsende (233) in demselben zu fixieren, einen ergriffenen Abschnitt (222), der sich von dem vorderen Verbindungsstück-Abschnitt (220) aus weg von dem vorderen Ende (225) erstreckt, und einen hinteren Stopfenabschnitt (223), der sich von dem ergriffenen Abschnitt (222) aus und distal von dem vorderen Verbindungsstück-Abschnitt (220) erstreckt und der eine innere Leitung (224) hat, die sich längs der Achse durch den ergriffenen Abschnitt (222) erstreckt, um mit dem hinteren Verbindungsende (233) verbunden zu sein, einschließt,
einen Zylinder (1), der eine innere umschließende Zylinderfläche (130) hat, welche die Achse umschließt und die einen Durchgang (11) in derselben definiert, wobei der Durchgang (11) eine hintere und eine vordere Öffnung (131, 132) hat, die in der Längsrichtung einander entgegengesetzt sind, wobei die innere umschließende Zylinderfläche (130) einen Abschnitt (133) mit größerem Durchmesser und einen Abschnitt (134) mit kleinerem Durchmesser einschließt, die nahe der hinteren bzw. der vorderen Öffnung (131, 132) angeordnet sind, wobei der Abschnitt (133) mit größerem Durchmesser einen Rückhaltebereich (137) hat, der in der Längsrichtung mit Zwischenraum zu dem Abschnitt (134) mit kleinerem Durchmesser angeordnet ist,
ein röhrenförmiges Greifelement (21), das in einer Verwendungsstellung dafür angeordnet ist, den ergriffenen Abschnitt (222) mit einer Haltekraft in einer Stellung der Unbeweglichkeit längs der Achse im Verhältnis zu dem Rückhaltebereich (137) zu halten,
einen röhrenförmigen Druckkolben (3), der dafür angeordnet ist, in dem Durchgang (11) längs des Abschnitts (133) mit größerem Durchmesser bewegt werden zu können,
wobei der Druckkolben (3) eine vordere geöffnete Stirnwand (321), die bewegt werden kann, um an das Greifelement (21) anzustoßen, um so den röhrenförmigen Druckkolben (3) in eine Vorentsorgungsstellung zu bringen, eine hintere Stirnwand (322), die entgegengesetzt zu der vorderen geöffneten Stirnwand (321) angeordnet ist und die sich von der hinteren Öffnung (131) aus nach außen erstreckt, um so von Hand betätigt werden zu können, und eine umschließende Zwischenwand (32), die zwischen die vordere geöffnete Stirnwand (321) und die hintere Stirnwand (322) geschaltet ist und eine Aufnahmeabteilung (31) definiert, hat,
wobei die intravenöse Katheter-Einführungseinrichtung ferner Folgendes umfasst:
einen röhrenförmigen Behälter (34), der ein Sockelende (343) und ein luftdurchlässiges Ende (344), die in der Längsrichtung mit Zwischenraum entfernt voneinander angeordnet sind, und ein röhrenförmiges Wandsegment (340), das zwischen des Sockelende (343) und des luftdurchlässige Ende (344) geschaltet ist, um eine Rückflusskammer (342) abzugrenzen, hat, wobei, wenn sich das röhrenförmige Greifelement (21) in der Verwendungsstellung befindet, der röhrenförmige Behälter (34) durch eine erste Reibungskraft in der Aufnahmeabteilung (31) zurückgehalten wird, wobei das Sockelende (343) bzw. das luftdurchlässige Ende (344) jeweils dem hinteren Stopfenabschnitt (223) und der hinteren Stirnwand (322) gegenüberliegen, um eine Luftverbindung zwischen der inneren Leitung (224) und der Rückflusskammer (342) herzustellen,
derart, dass, wenn sich der röhrenförmige Druckkolben (3) in der Vorentsorgungsstellung befindet, das Sockelende (343) in Eingriff mit dem hinteren Stopfenabschnitt (223) gebracht ist,
derart, dass, wenn das Greifelement (21) auf Grund einer Vorwärtsbewegung der vorderen geöffneten Stirnwand (321) gegen die Haltekraft vorwärtsgeschoben wird, der ergriffene Abschnitt (222) von dem Greifelement (21) gelöst wird, um eine axiale Bewegung des ergriffenen Abschnitts (222) im Verhältnis zu dem Rückhaltebereich (137) zu ermöglichen, und
derart, dass, wenn der ergriffene Abschnitt (222) von dem Greifelement (21) gelöst wird, eine fortgesetzte Vorwärtsbewegung der vorderen geöffneten Stirnwand (321) gegen die erste Reibungskraft auf Grund einer Vorspannkraft zu einer Bewegung des Nadelsitzes (22) zusammen mit dem röhrenförmigen Behälter (34), durch den Eingriff des hinteren Stopfenabschnitts (223) mit dem Sockelende (343), zu der hinteren Stirnwand hin führen wird, um so den Nadelsitz (22) und die Nadelkanüle (23) in eine Entsorgungsstellung zu bringen, worin das Spitzenende (232) der Nadelkanüle (23) in den Durchgang (11) eingezogen ist und worin sich das Sockelende (343) näher an der hinteren Stirnwand (332) befindet als in der Verwendungsstellung, und
ein Vorspannelement (4), das dafür angeordnet ist, die Vorspannkraft bereitzustellen.

2. Intravenöse Katheter-Einführungseinrichtung nach Anspruch 1, **dadurch gekennzeichnet, dass** der Abschnitt (134) mit kleinerem Durchmesser einen Eintrittsbereich (15), der dafür konfiguriert ist, den vorderen Verbindungsstück-Abschnitt (220) zu umschließen, und einen Übergangsbereich (17), der sich von dem Eintrittsbereich (15) aus zu der vorderen Öffnung (132) hin erstreckt, einschließt, wobei das röhrenförmige Greifelement (21) so angeordnet ist, dass es sich in Rückhalteeingriff mit dem Rückhaltebereich (137) des Abschnitts (133) mit größerem Durchmesser und in Greifeingriff mit dem ergriffenen Abschnitt (222) des Nadelsitzes (22) befindet, durch eine zweite bzw. eine dritte Reibungskraft, die in radialen Richtungen zusammenwirken, um als die Haltekraft zu dienen derart, dass, sobald auf Grund der Vorwärtsbewegung des Greifelements (21) und der vorderen geöffneten Stirnwand (321) eine axiale Bewegung des ergriffenen Abschnitts (222) im Verhältnis zu dem Rückhaltebereich (137) beginnt, der vordere Verbindungsstück-Abschnitt (220) bewegt wird, um von dem Eintrittsbereich (15) zu dem Übergangsbereich (17) hinüberzugehen, um so zu ermöglichen, dass vor dem Ausrücken des ergriffenen Abschnitts (222) von dem röhrenförmigen Greifelement (21) in der Vorentsorgungsstellung das röhrenförmige Greifelement (21) von dem Rückhaltebereich (137) ausgerückt wird.

3. Intravenöse Katheter-Einführungseinrichtung nach Anspruch 2, **dadurch gekennzeichnet, dass** der vordere Verbindungsstück-Abschnitt (220) des Nadelsitzes (22) eine Absatzfläche (221) hat, die rückwärts von dem vorderen Ende (225) angeordnet ist und die zu der vorderen Öffnung (132) zeigt, wobei der Abschnitt (134) mit kleinerem Durchmesser ferner eine innere ringförmige Widerlagerfläche (16) einschließt, die durch den Übergangsbereich (17) mit Zwischenraum entfernt von der Absatzfläche (221) angeordnet ist derart, dass auf Grund des Anstoßens der Absatzfläche (221) an der inneren ringförmigen Widerlagerfläche (16) eine Vorwärtsbewegung des Greifelements (21) und der vorderen geöffneten Stirnwand (321) gegen die dritte Reibungskraft zu einem Ausrücken des ergriffenen Abschnitts (222) von dem Greifelement (21) führt.

4. Intravenöse Katheter-Einführungseinrichtung nach Anspruch 3, **dadurch gekennzeichnet, dass** der Zylinder (1) eine vordere Stirnwand (139) hat, welche die vordere Öffnung (132) definiert, wobei das Vorspannelement (4) die Form einer Schraubenfeder (4) hat, die das vordere Segment (231) der Nadelkanüle (23) umschließt und die zwischen der vorderen Stirnwand (139) und der Absatzfläche (221) des Nadelsitzes (22) zusammengedrückt wird.

5. Intravenöse Katheter-Einführungseinrichtung nach Anspruch 1, **dadurch gekennzeichnet, dass** das röhrenförmige Wandsegment (340) des röhrenförmigen Behälters (34) eine äußere ringförmige Widerlagerfläche (345), angrenzend an das luftdurchlässige Ende (344), hat, wobei das Vorspannelement (4) die Form einer Schraubenfeder (4) hat, die das röhrenförmige Wandsegment (340) umschließt und die zwischen der vorderen geöffneten Stirnwand (321) und der äußeren ringförmigen Widerlagerfläche (345) zusammengedrückt wird.

6. Intravenöse Katheter-Einführungseinrichtung nach Anspruch 1, **dadurch gekennzeichnet, dass** das Vorspannelement (4) die Form einer Schraubenfeder (4) hat, die in der Aufnahmeabteilung (31) aufgenommen wird und die ein befestigtes Ende (43), das an der umschließenden Zwischenwand (32) angrenzend an die hintere Stirnwand (322) befestigt ist, und ein gespanntes Ende (44), das an dem röhrenförmigen Wandsegment (340) des röhrenförmigen Behälters (34) befestigt ist, hat, um so in der Verwendungsstellung gespannt zu bleiben.

7. Intravenöse Katheter-Einführungseinrichtung nach Anspruch 1, **dadurch gekennzeichnet, dass** die hintere Stirnwand (322) des röhrenförmigen Druckkolbens (3) eine Zugangsöffnung zum Einsetzen des röhrenförmigen Behälters (34) in die Aufnahmeabteilung (31) definiert, wobei die Einrichtung ferner eine Endkappe (37) umfasst, die abnehmbar an der hinteren Stirnwand (322) angebracht ist, um die Zugangsöffnung zu verschließen, wobei das Vorspannelement (4) die Form einer Schraubenfeder (4) hat, die in der Aufnahmeabteilung (31) aufgenommen wird und die ein befestigtes Ende (43), das an der Endkappe (37) befestigt ist, und ein gespanntes Ende (44), das an dem röhrenförmigen Wandsegment (340) des röhrenförmigen Behälters (34) befestigt ist, hat, um so in der Verwendungsstellung gespannt zu bleiben.

8. Intravenöse Katheter-Einführungseinrichtung nach Anspruch 1, ferner **gekennzeichnet durch** ein luftdurchlässiges Element (35), das aus einem porösen Material hergestellt ist und das integral mit dem luftdurchlässigen Ende (344) geformt ist, um so zu verhindern, dass Blut aus der Rückflusskammer (342) heraussickert.

9. Intravenöse Katheter-Einführungseinrichtung nach Anspruch 1, **dadurch gekennzeichnet, dass** der röhrenförmige Druckkolben (3) ein verformbares Abdichtungselement (36) hat, das dafür konfiguriert ist, die vordere geöffnete Stirnwand (321) zu umschließen, und das sich in luftdichtem Gleiteingriff mit dem Abschnitt (133) mit größerem Durchmesser befindet.

10. Intravenöse Katheter-Einführungseinrichtung nach Anspruch 1, **dadurch gekennzeichnet, dass** der röhrenförmige Druckkolben (3) einen Auslass (33) hat, der die Aufnahmeabteilung (31) mit der Umgebungsluft verbindet und der stromabwärts von dem luftdurchlässigen Ende (344) angeordnet ist.

11. Intravenöse Katheter-Einführungseinrichtung nach Anspruch 10, **dadurch gekennzeichnet, dass** der Auslass (33) in der umschließenden Zwischenwand (32), angrenzend an die hintere Stirnwand (322), geformt ist, um das Verschließen durch einen Finger eines Benutzers zu erleichtern, wenn der Benutzer den röhrenförmigen Druckkolben (3) ergreift und bewegt.

12. Intravenöse Katheter-Einführungseinrichtung nach Anspruch 10, **dadurch gekennzeichnet, dass** die hintere Stirnwand (322) des röhrenförmigen Druckkolbens (3) eine Zugangsöffnung zum Einsetzen des röhrenförmigen Behälters (34) in die Aufnahmeabteilung (31) definiert, wobei die Einrichtung ferner eine Endkappe (37) umfasst, die abnehmbar an der hinteren Stirnwand (322) angebracht ist, um die Zugangsöffnung zu verschließen, wobei der Auslass (33) in der Endkappe (37) geformt ist.

13. Intravenöse Katheter-Einführungseinrichtung nach Anspruch 10, **dadurch gekennzeichnet, dass** der Auslass (33) in der hinteren Stirnwand (322) des röhrenförmigen Druckkolbens (3) geformt ist.

14. Intravenöse Katheter-Einführungseinrichtung nach Anspruch 13, ferner **gekennzeichnet durch** eine Abdeckplatte (38), die an dem röhrenförmigen Druckkolben (3) angeordnet ist und die im Verhältnis zu dem röhrenförmigen Druckkolben (3) zwischen einer Verschluss- und einer Öffnungsstellung bewegt werden kann, wobei die Abdeckplatte (38) an der hinteren Stirnwand (322) eingerückt und von derselben ausgerückt wird, um den Auslass (33) zu öffnen bzw. zu schließen.

15. Intravenöse Katheter-Einführungseinrichtung nach Anspruch 13, **dadurch gekennzeichnet, dass** die umschließende Zwischenwand (32) des röhrenförmigen Druckkolbens (3) eine ringförmige Sperre (327) hat, die angrenzend an die hintere Stirnwand (322) geformt ist derart, dass das luftdurchlässige Ende (344) des röhrenförmigen Behälters (34) durch die ringförmige Sperre (327) angehalten wird, wenn der röhrenförmige Behälter (34) zu der Entsorgungsstellung bewegt wird.

16. Intravenöse Katheter-Einführungseinrichtung nach Anspruch 1, ferner **gekennzeichnet durch**
ein Katheterverbindungsstück (24), das in demselben eine Leitung (241) definiert, die ein Erstrecken des vorderen Segments (231) der Nadelkanüle (23) **durch** dieselbe ermöglicht, und
einen röhrenförmigen Katheter (25), der ein proximales Segment (251), das in die Leitung (241) eingesetzt wird, und ein distales Segment (252), das sich von dem proximalen Segment (251) aus erstreckt und welches das vordere Segment (231) der Nadelkanüle (23) umschließt und umhüllt, hat, während ermöglicht wird, dass das Spitzenende (232) vorwärts von dem distalen Segment (252) aus vorspringt, um die Haut eines Patienten zu durchstechen.

17. Intravenöse Katheter-Einführungseinrichtung nach Anspruch 1, **dadurch gekennzeichnet, dass** der röhrenförmige Druckkolben (3) bzw. der Abschnitt (133) mit größerem Durchmesser einen ringförmigen Vorsprung (325) und eine ringförmige Aussparung (136) haben, die derart konfiguriert sind, dass, sobald der röhrenförmige Druckkolben (3) in die Vorentsorgungsstellung gebracht wird, der ringförmige Vorsprung (325) und die ringförmige Aussparung (136) satt in Eingriff miteinander gebracht werden, um so ein Rückwärtsziehen des röhrenförmigen Druckkolbens (3) für eine Wiederverwendung zu verhindern.

18. Intravenöse Katheter-Einführungseinrichtung nach Anspruch 1, **dadurch gekennzeichnet, dass** die umschließende Zwischenwand (32) des röhrenförmigen Druckkolbens (3) mehrere Rippen (324) hat, die an einer Außenfläche derselben und angrenzend an die hintere Stirnwand (322) geformt sind und die dafür angeordnet sind, an der hinteren Öffnung (131) an den Zylinder (1) anzustoßen, wenn sich der röhrenförmige Druckkolben (3) in der Entsorgungsstellung befindet.

## Revendications

1. Dispositif d'introduction d'un cathéter intraveineux, comprenant :
une canule d'aiguille (23), comportant un segment avant (231) se terminant au niveau d'une extrémité de pointe (232), et une extrémité de connexion arrière (233) opposée audit segment avant (231) le long de l'axe, dans une direction longitudinale ;
un siège d'aiguille tubulaire (22), englobant une partie de moyeu avant (220) s'étendant le long de l'axe et se terminant au niveau d'une extrémité avant (225), et agencée pour y fixer ladite extrémité de connexion arrière (233), une partie à préhension (222) s'étendant de ladite partie de moyeu avant (220) à l'écart de ladite extrémité avant (225), et une partie de bouchon arrière (223) s'étendant à partir de ladite partie à préhension (222) et distale par rapport à ladite partie de moyeu avant (220), et comportant un conduit interne (224) s'étendant à partir de ladite partie à préhension (222) le long de l'axe en vue d'établir une communication avec ladite extrémité de connexion arrière (233) ;
un cylindre (1), comportant une surface de cylindre environnante interne (130), entourant l'axe, et y définissant un passage (11), ledit passage (11) comportant des ouvertures arrière et avant (131, 132) opposées l'une à l'autre dans la direction longitudinale, ladite surface de cylindre environnante interne (130) englobant une partie à diamètre accru (133) et une partie à diamètre réduit (134), agencées respectivement près desdites ouvertures arrière et avant (131, 132), ladite partie à diamètre accru (133) comportant une zone de retenue (137) espacée de ladite partie à diamètre réduit (134) dans la direction longitudinale ;
un élément de préhension tubulaire (21), agencé dans une position de service de sorte à retenir, par une force de retenue, ladite partie à préhension (222) dans une position d'immobilité le long de l'axe par rapport à ladite zone de retenue (137) ; et
un piston tubulaire (3) agencé de sorte à pouvoir être déplacé dans ledit passage (11) le long de ladite partie à diamètre accru (133) ; **caractérisé en ce que**
ledit piston (3) comporte une paroi d'extrémité avant ouverte (321), pouvant être déplacée pour buter contre ledit élément de préhension (21), pour placer ledit piston tubulaire (3) dans une position occupée avant son évacuation, une paroi d'extrémité arrière (322) agencée en un point opposé à ladite paroi d'extrémité avant ouverte (321) et s'étendant vers l'extérieur de ladite ouverture arrière (131), de sorte à pouvoir être actionnée manuellement, et une paroi intermédiaire environnante (32) agencée entre ladite paroi d'extrémité avant ouverte (321) et ladite paroi d'extrémité arrière (322) et définissant un compartiment de réception (31) ;
ledit dispositif d'introduction du cathéter intraveineux comprenant en outre :
un réceptacle tubulaire (34) comportant une extrémité de douille (343) et une extrémité perméable à l'air (344) espacées l'une de l'autre dans la direction longitudinale, et un segment de paroi tubulaire (340) agencé entre ladite extrémité de douille (343) et ladite extrémité perméable à l'air (344) pour confiner une chambre de reflux (342), dans lequel, ledit élément de préhension tubulaire (21) se trouvant dans la position de service, ledit réceptacle tubulaire (34) est retenu dans ledit compartiment de réception (31) par une première force de frottement, ladite extrémité de douille (343) et ladite extrémité perméable à l'air (344) faisant respectivement face à ladite partie de bouchon (223) et à ladite paroi d'extrémité arrière (322) pour établir une communication d'air entre ledit conduit interne (224) et ladite chambre de reflux (342) ;
de sorte que lorsque ledit piston tubulaire (3) se trouve dans la position occupée avant son évacuation, ladite extrémité de douille (343) est engagée dans ladite partie de bouchon arrière (223) ;
de sorte que lorsque ledit élément de préhension (21) est poussé vers l'avant par suite d'un déplacement vers l'avant de ladite paroi d'extrémité ouverte (321), contre la force de retenue, ladite partie à préhension (222) est dégagée dudit élément de préhension (21) pour permettre un déplacement axial de ladite partie à préhension (222) par rapport à ladite zone de retenue (137) ; et
de sorte que lorsque ladite partie à préhension (22) est dégagée dudit élément de préhension (21) un déplacement continu de ladite paroi d'extrémité avant ouverte (321), contre la force de frottement, entraîne un déplacement dudit siège d'aiguille (22), ensemble avec ledit réceptacle tubulaire (34), par suite de l'engagement de ladite partie de bouchon arrière (223) dans ladite extrémité de douille (343), vers ladite paroi d'extrémité arrière (322), par l'intermédiaire d'une force de poussée, pour placer ledit siège d'aiguille (22) et ladite canule d'aiguille (23) dans une position d'évacuation, dans laquelle ladite extrémité de pointe (232) de ladite canule d'aiguille (23) est rétractée dans ledit passage (11), ladite extrémité de douille (343) étant plus proche de ladite paroi d'extrémité arrière (322) que dans la position de service ; et
un élément poussoir (4), destiné à produire ladite force de poussée.

2. Dispositif d'introduction d'un cathéter intraveineux selon la revendication 1, **caractérisé en ce que** ladite partie à diamètre réduit (134) englobe une région d'entrée (15), configurée de sorte à entourer ladite partie de moyeu avant (220), et une région de transition (17) s'étendant de ladite région d'entrée (15) vers ladite ouverture avant (132), ledit élément de préhension tubulaire (21) étant agencé de sorte à être engagé dans ladite zone de retenue (137) de ladite partie à diamètre accru (133) et à retenir celle-ci, et à être respectivement engagé par préhension dans ladite partie à préhension (222) dudit siège d'aiguille (122), par l'intermédiaire de deuxième et troisième forces de frottement, coopérant dans des directions radiales pour servir de force de retenue, de sorte que lorsque ladite partie à préhension (222) commence son déplacement axial par rapport à ladite zone de retenue (137), par suite du déplacement vers l'avant dudit élément de préhension (21) et de ladite paroi d'extrémité avant ouverte (321), ladite partie de moyeu avant (220) est déplacée pour passer de ladite région d'entrée (15) vers ladite région de transition (17), pour permettre le dégagement dudit élément de préhension tubulaire (21) de ladite zone de retenue (137) avant le dégagement de ladite partie à préhension (222) dudit élément de préhension tubulaire (21) dans la position occupée avant son évacuation.

3. Dispositif d'introduction d'un cathéter intraveineux selon la revendication 2, **caractérisé en ce que** ladite partie de moyeu avant (220) dudit siège d'aiguille (22) comporte une surface d'épaulement (221) agencée vers l'arrière de ladite extrémité frontale (225) et orientée vers ladite ouverture avant (132), ladite partie à diamètre réduit (134) englobant en outre une surface de butée annulaire interne (16) espacée de ladite surface d'épaulement (221) par ladite région de transition (17), de sorte que par suite de la butée de ladite surface d'épaulement (221) contre ladite surface de butée annulaire interne (16), le déplacement vers l'avant dudit élément de préhension (21) et de ladite paroi d'extrémité avant ouverte (321) entraîne le dégagement de ladite partie à préhension (222) dudit élément de préhension (21), contre la troisième force de frottement.

4. Dispositif d'introduction d'un cathéter intraveineux selon la revendication 3, **caractérisé en ce que** ledit cylindre (1) comporte une paroi d'extrémité avant (139), définissant ladite ouverture avant (132), ledit élément poussoir (4) ayant la forme d'un ressort hélicoïdal (4) entourant ledit segment avant (231) de ladite canule d'aiguille (23), et étant comprimé entre ladite paroi d'extrémité avant (139) et ladite surface d'épaulement (221) dudit siège d'aiguille (22).

5. Dispositif d'introduction d'un cathéter intraveineux selon la revendication 1, **caractérisé en ce que** ledit segment de paroi tubulaire (340) dudit réceptacle tubulaire (34) comporte une surface de butée annulaire externe (345) adjacente à ladite extrémité perméable à l'air (344), ledit élément poussoir (4) ayant la forme d'un ressort hélicoïdal (4) entourant ledit segment de paroi tubulaire (340) et comprimé entre ladite paroi d'extrémité avant ouverte (321) et ladite surface de butée annulaire externe (345).

6. Dispositif d'introduction d'un cathéter intraveineux selon la revendication 1, **caractérisé en ce que** ledit élément poussoir (4) a la forme d'un ressort hélicoïdal (4), reçu dans ledit compartiment de réception (31) et comportant une extrémité fixée (43), fixée sur ladite paroi environnante intermédiaire (32) près de ladite paroi d'extrémité arrière (322), et une extrémité tendue (44) fixée sur ledit segment de paroi tubulaire (340) dudit réceptacle tubulaire (34), de sorte à rester tendue dans la position de service.

7. Dispositif d'introduction d'un cathéter intraveineux selon la revendication 1, **caractérisé en ce que** ladite paroi d'extrémité arrière (322) dudit piston tubulaire (3) définit une ouverture d'accès en vue de l'insertion dudit réceptacle tubulaire (34) dans ledit compartiment de réception (31), ledit dispositif comprenant en outre un capuchon d'extrémité (37) monté de manière amovible sur ladite paroi d'extrémité arrière (322) pour fermer ladite ouverture d'accès, ledit élément poussoir (4) ayant la forme d'un ressort hélicoïdal (4) reçu dans ledit compartiment de réception (31) et comportant une extrémité fixée (43), fixée sur ledit capuchon d'extrémité (37), et une extrémité tendue (44) fixée sur ledit segment de paroi tubulaire (340) dudit réceptacle tubulaire (34), de sorte à rester tendue dans la position de service.

8. Dispositif d'introduction d'un cathéter intraveineux selon la revendication 1, **caractérisé en outre par** un élément perméable à l'air (35), composé d'un matériau poreux, et formé d'une seule pièce avec ladite extrémité perméable à l'air (344), pour empêcher un écoulement du sang hors de ladite chambre de reflux (342).

9. Dispositif d'introduction d'un cathéter intraveineux selon la revendication 1, **caractérisé en ce que** ledit piston tubulaire (3) comporte un élément d'étanchéité déformable (36), configuré de sorte à entourer ladite paroi d'extrémité avant ouverte (321) et engagé de manière coulissante et étanche à l'air dans ladite partie à diamètre accru (133).

10. Dispositif d'introduction d'un cathéter intraveineux selon la revendication 1, **caractérisé en ce que** ledit piston tubulaire (3) comporte une sortie (33), établissant une communication entre ledit compartiment de réception (31) et l'air ambiant, et agencée en aval de ladite extrémité perméable à l'air (344).

11. Dispositif d'introduction d'un cathéter intraveineux selon la revendication 10, **caractérisé en ce que** ladite sortie (33) est formée dans ladite paroi environnante intermédiaire (32), près de ladite paroi d'extrémité arrière (322), pour faciliter la fermeture par le doigt d'un utilisateur lorsque l'utilisateur saisit ledit piston tubulaire (3) et déplace celui-ci.

12. Dispositif d'introduction d'un cathéter intraveineux selon la revendication 10, **caractérisé en ce que** ladite paroi d'extrémité arrière (322) dudit piston tubulaire (3) définit une ouverture d'accès en vue de l'insertion dudit réceptacle tubulaire (34) dans ledit compartiment de réception (31), ledit dispositif comprenant en outre un capuchon d'extrémité (37), monté de manière amovible sur ladite paroi arrière (322) pour fermer ladite ouverture d'accès, ladite sortie (33) étant formée dans ledit capuchon d'extrémité (37).

13. Dispositif d'introduction d'un cathéter intraveineux selon la revendication 10, **caractérisé en ce que** ladite sortie (33) est formée dans ladite paroi d'extrémité arrière (322) dudit piston tubulaire (3).

14. Dispositif d'introduction d'un cathéter intraveineux selon la revendication 13, **caractérisé en outre par** une plaque de couverture (38) agencée sur ledit piston tubulaire (3) et pouvant être déplacée par rapport audit piston tubulaire (3) entre des positions de fermeture et d'ouverture, dans lesquelles ladite plaque de couverture (38) s'engage dans ladite paroi d'extrémité arrière (322) et se dégage de celle-ci pour fermer et ouvrir respectivement ladite sortie (33).

15. Dispositif d'introduction d'un cathéter intraveineux selon la revendication 13, **caractérisé en ce que** ladite paroi environnante intermédiaire (32) dudit piston tubulaire (3) comporte une barrière annulaire (327) formée près de ladite paroi d'extrémité arrière (322), de sorte que ladite extrémité perméable à l'air (344) dudit réceptacle tubulaire (34) est arrêtée par ladite barrière annulaire (327) lors du déplacement dudit réceptacle tubulaire (34) vers la position d'évacuation.

16. Dispositif d'introduction d'un cathéter intraveineux selon la revendication 1, **caractérisé en outre par** :
un moyeu de cathéter (24) définissant un conduit (241) permettant l'extension dudit segment avant (231) de ladite canule d'aiguille (23) à travers celui-ci ; et
un cathéter tubulaire (25) comportant un segment proximal (251) inséré dans ledit conduit (241), et un segment distal (252) s'étendant à partir dudit segment proximal (251) et entourant et protégeant ledit segment avant (231) de ladite canule d'aiguille (23), tout en permettant le débordement de ladite extrémité de pointe (232) vers l'avant dudit segment distal (252) pour percer la peau d'un patient.

17. Dispositif d'introduction d'un cathéter intraveineux selon la revendication 1, **caractérisé en ce que** ledit piston tubulaire (3) et ladite partie à diamètre accru (133) comportent respectivement une saillie annulaire (325) et un évidement annulaire (136) configurés de sorte que lorsque ledit piston tubulaire (3) est placé dans la position occupée avant son évacuation, ladite saillie annulaire (325) et ledit évidement annulaire (136) sont engagés fermement l'un dans l'autre, pour empêcher une traction vers l'arrière dudit piston tubulaire (3) en vue d'une réutilisation.

18. Dispositif d'introduction d'un cathéter intraveineux selon la revendication 1, **caractérisé en ce que** ladite paroi environnante intermédiaire (32) dudit piston tubulaire (3) comporte plusieurs nervures (324) formées sur une de ses surfaces externes, et adjacentes à ladite paroi d'extrémité arrière (322), agencées de sorte à buter contre ledit cylindre (1) au niveau de ladite ouverture arrière (131) lorsque ledit piston tubulaire (3) se trouve dans la position d'évacuation.
